# EUROPEAN PATENT SPECIFICATION

(11) **EP 4 257 162 B1**
(45) Date of publication and mention of the grant of the patent: **08.07.2026**
(21) Application number: 23166497.0
(22) Date of filing: 04.04.2023
(51) Int. Cl.: A61M 1/36, A61M 1/38

(54) **SYSTEMS AND METHODS FOR IMPLEMENTING DIFFERENT VERSIONS OF A BLOOD SEPARATION PROCEDURE**
SYSTEME UND VERFAHREN ZUR IMPLEMENTIERUNG VERSCHIEDENER VERSIONEN EINES BLUTTRENNUNGSVERFAHRENS
SYSTÈMES ET PROCÉDÉS POUR METTRE EN OEUVRE DIFFÉRENTES VERSIONS D'UNE PROCÉDURE DE SÉPARATION DU SANG

(30) Priority: 06.04.2022 US 202263327869 P; 03.06.2022 US 202263348839 P
(43) Date of publication of application: 11.10.2023
(73) Proprietor: Fenwal, Inc., Lake Zurich, IL 60047 (US)
(72) Inventor: PLANAS, Samantha M., Lake Zurich, 60047 (US); MIN, Kyungyoon, Lake Zurich, 60047 (US); PATEL, Amit J., Lake Zurich, 60047 (US); GNIADEK, Thomas, Lake Zurich, 60047 (US)
(74) Representative: Maikowski & Ninnemann Patentanwälte Partnerschaft mbB

(56) References cited:
- US-A1- 2004 199 098
- US-A1- 2006 052 949

## Description

### Background

### Field of the Disclosure

The present disclosure relates to blood separation systems configured to perform two different versions of one blood separation procedure.

### Description of Related Art

Various blood processing systems now make it possible to collect particular blood constituents, instead of whole blood, from a blood source such as a container of previously collected blood or other living or non-living source. Blood is typically separated into its constituents (e.g., red cells, platelets, and plasma) through centrifugation, such as in the AMICUS^{®} separator from Fenwal, Inc. of Lake Zurich, Illinois, which is an affiliate of Fresenius Kabi AG of Bad Homburg, Germany, or other centrifugal separation devices, or a spinning membrane-type separator, such as the AUTOPHERESIS-C^{®} and AURORA^{®} devices from Fenwal, Inc. In such systems, whole blood is drawn from a blood source, the particular blood component or constituent is separated, removed, and collected, and the remaining blood constituents are returned to the blood source.

Removing only particular constituents is advantageous when the blood source is a human donor because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations. The invention is defined by the appended claims.

Herein described although not part of the invention is a method for implementing a blood separation procedure. The method includes selecting a first blood separation device configured to execute a first version of a blood separation procedure and a second blood separation device configured to execute a second version of the blood separation procedure or a single blood separation device configured to execute the first and second versions of the blood separation procedure. It is determined whether a blood source is qualified for the first version of the blood separation procedure or the second version of the blood separation procedure and/or whether a blood source is not qualified for the first version of the blood separation procedure or the second version of the blood separation procedure. The first version of the blood separation procedure is implemented for the blood source upon determining that the blood source is qualified for the first version of the blood separation procedure and/or upon determining that the blood source is not qualified for the second version of the blood separation procedure. The second version of the blood separation procedure is instead implemented for the blood source upon determining that the blood source is qualified for the second version of the blood separation procedure and/or upon determining that the blood source is not qualified for the first version of the blood separation procedure.

The invention relates to a blood separation device which includes a pump system, a clamp system, a separation actuator, and a controller. The controller is programmed with first and second versions of a blood separation procedure in which the pump system, the clamp system, and the separation actuator are actuated to draw blood from a blood source, separate the blood into at least two blood components, and convey at least a portion of one of said blood components to a recipient. The controller is configured to determine or be instructed that the blood source is qualified for the first version of the blood separation procedure or the second version of the blood separation procedure and/or determine or be instructed that the blood source is not qualified for the first version of the blood separation procedure or the second version of the blood separation procedure. The controller implements the first version of the blood separation procedure for the blood source upon determining or being instructed that the blood source is qualified for the first version of the blood separation procedure and/or upon determining or being instructed that the blood source is not qualified for the second version of the blood separation procedure. The controller instead implements the second version of the blood separation procedure for the blood source upon determining or being instructed that the blood source is qualified for the second version of the blood separation procedure and/or upon determining or being instructed that the blood source is not qualified for the first version of the blood separation procedure.

In another aspect, a blood separation device includes a pump system, a clamp system, a separation actuator, and a controller. The controller is configured to actuate the pump system, the clamp system, and the separation actuator to execute a blood separation procedure in which a volume of blood having a volume of red blood cells is present in a fluid flow circuit. The controller is further configured to be instructed of a maximum percentage and/or maximum volume of a total blood volume of a blood source that may be present in the fluid flow circuit at any one time during said blood separation procedure and/or a maximum percentage and/or maximum volume of a pre-procedure red blood cell volume of the blood source that may be present in the fluid flow circuit at any one time during said blood separation procedure.

Also described but not part of the invention, a computer-implemented method is provided for executing a blood separation procedure using a first blood separation device configured to execute a first version of a blood separation procedure and a second blood separation device configured to execute a second version of the blood separation procedure or a single blood separation device configured to execute the first and second versions of the blood separation procedure. The method includes determining whether a blood source is qualified for the first version of the blood separation procedure or the second version of the blood separation procedure and/or determining whether a blood source is not qualified for the first version of the blood separation procedure or the second version of the blood separation procedure. The first version of the blood separation procedure is initiated (or an operator is prompted to initiate the first version of the procedure) for the blood source upon determining that the blood source is qualified for the first version of the blood separation procedure and/or upon determining that the blood source is not qualified for the second version of the blood separation procedure. The second version of the blood separation procedure is initiated (or an operator is prompted to initiate the second version of the procedure) for the blood source upon determining that the blood source is qualified for the second version of the blood separation procedure and/or upon determining that the blood source is not qualified for the first version of the blood separation procedure.

### Brief Description of the Drawings

Fig. 1 is a front perspective view of an exemplary blood separation device according to an aspect of the present disclosure;
Fig. 2 is a rear perspective view of the blood separation device of Fig. 1, with a rear door thereof in an open position;
Fig. 3 is a front perspective view of the blood separation device of Fig. 1, with a fluid flow circuit associated therewith to constitute a blood separation system;
Fig. 4 is a front perspective view of a blood separation chamber of the fluid flow circuit of Fig. 3, with a portion thereof broken away for illustrative purposes;
Fig. 5 is a schematic view of the blood separation system of Fig. 3, in a blood draw mode;
Fig. 6 is a schematic view of the blood separation system of Fig. 3, in a blood component return mode;
Fig. 7 is a schematic view of the fluid flow circuit and fluid processing system of Fig. 3, in a replacement fluid return mode; and
Figs. 8A, 8B, 9A, and 9B illustrate exemplary screens that may be presented on a display or data entry device of the blood separation device of Fig. 1.

### Description of the Illustrated Embodiments

The embodiments disclosed herein are for the purpose of providing an exemplary description of the present subject matter. They are, however, only exemplary, and the present subject matter may be embodied in various forms. Therefore, the scope of the invention is solely defined by the appended claims.

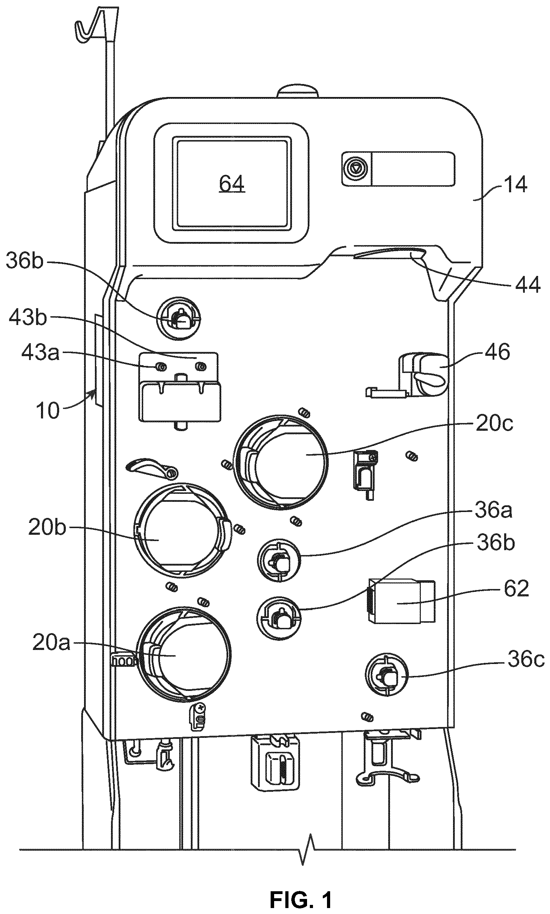

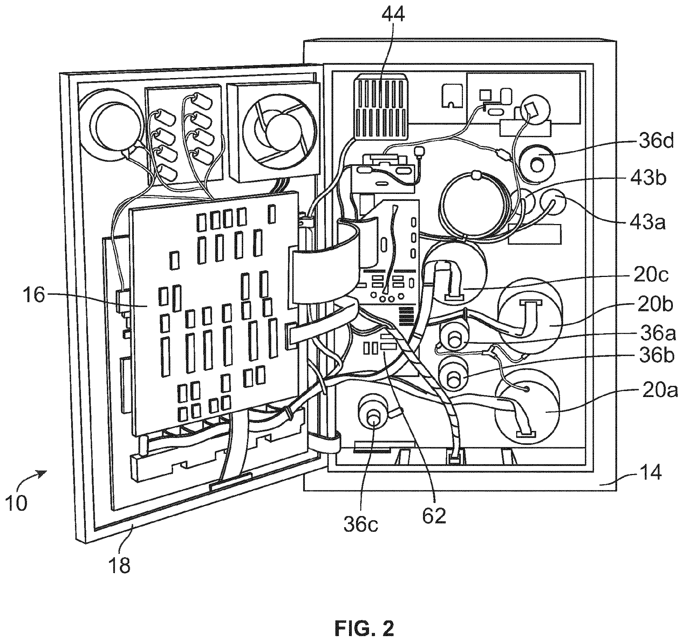

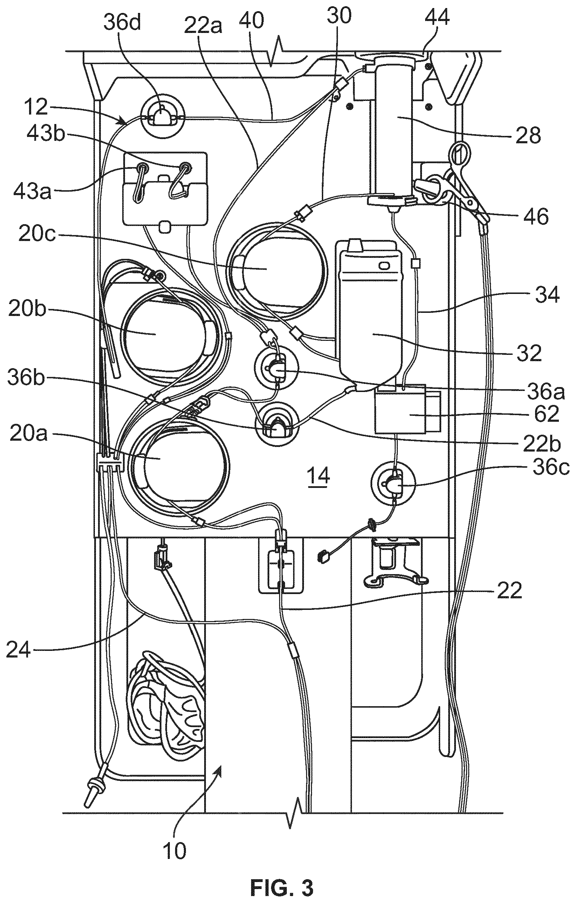

According to an aspect of the present disclosure, a durable or reusable blood separation device is used in combination with a separate fluid flow circuit (which may be disposable) to separate blood into two or more constituents. Figs. 1 and 2 illustrate an exemplary blood separation device 10, while Fig. 3 illustrates an exemplary fluid flow circuit 12 mounted onto the blood separation device 10 (with the combination referred to herein as a "blood separation system"). It should be understood that the illustrated blood separation device 10 and fluid flow circuit 12 are merely exemplary of such devices and circuits and that differently configured blood separation devices and fluid flow circuits may be provided.

The device 10 of Fig. 1 is configured for processing whole blood, but it may be used to process any other plasma-containing fluid to separate plasma from concentrated fluid (which may be red cell concentrate or packed red cells when the plasma-containing fluid constitutes whole blood). The plasma-containing fluid may come from any fluid source, which may include a living donor or patient (e.g., a human blood donor) or a non-living source (e.g., a blood bag or fluid container).

The illustrated device 10 includes a cabinet or housing 14, with several components positioned outside of the cabinet 14 (e.g., associated with a front wall or surface or panel of the cabinet 14) and additional components (including a central processing unit or controller 16) and interconnects positioned inside of the cabinet 14, which may be accessed by opening a rear door 18 of the device 10, as shown in Fig. 2. It should be understood that the illustrated components and the location of the components is merely exemplary, and that additional or different components and different component arrangements may be incorporated into the device.

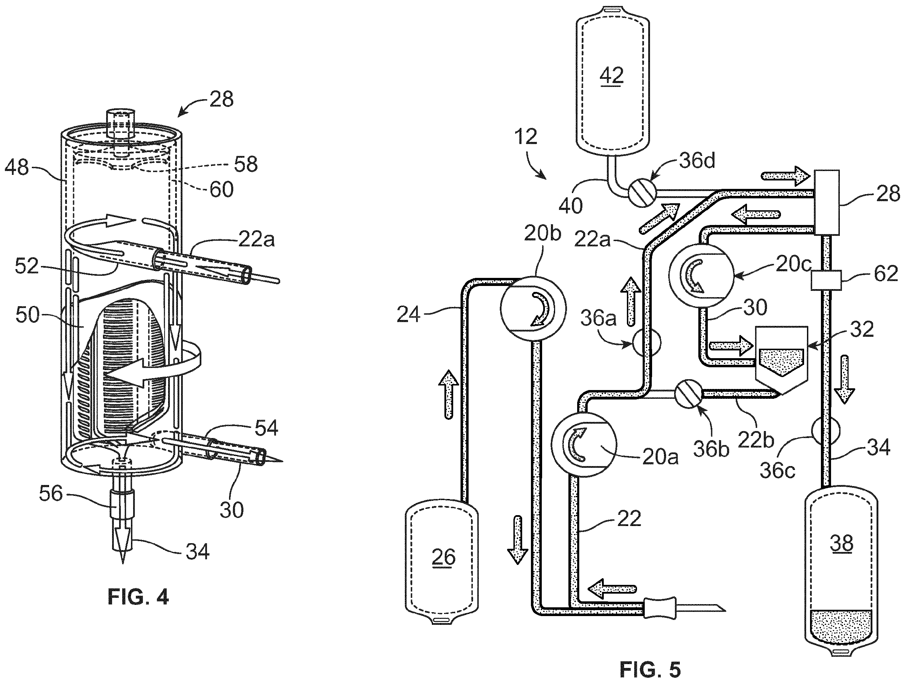

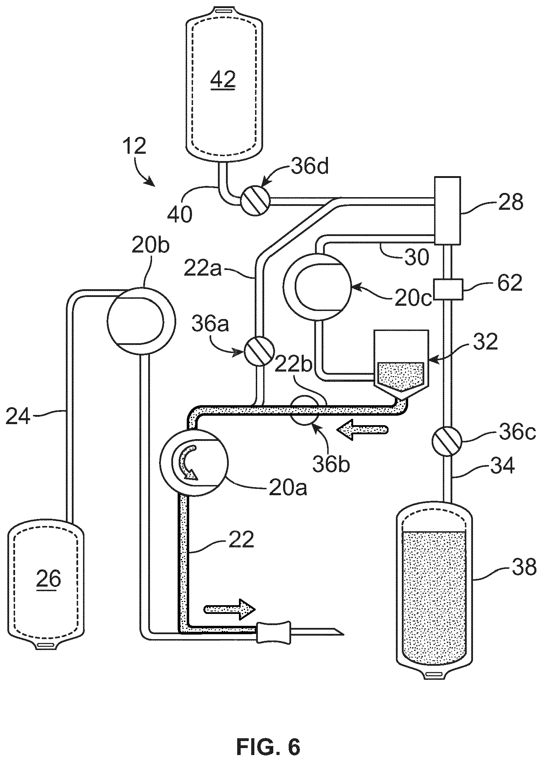

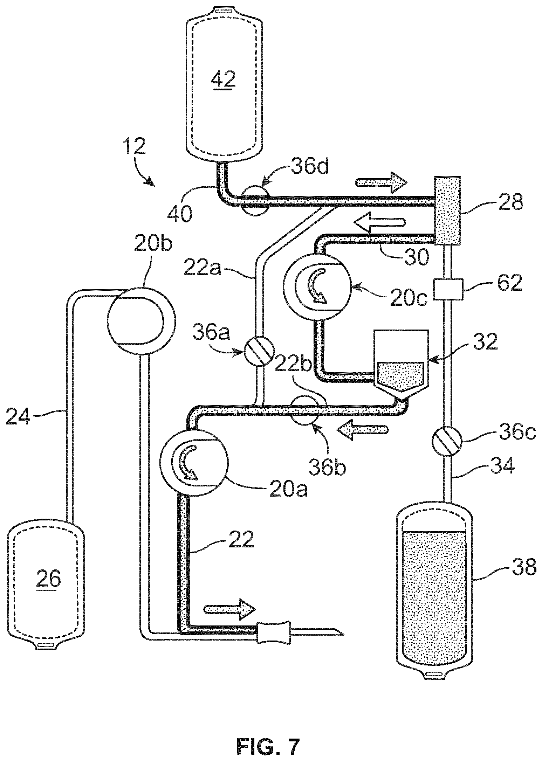

Among the components positioned on the outside of the cabinet 14, one or more pumps or pump stations 20a-20c (collectively referred to herein as a "pump system") may be provided, with the pumps 20a-20c configured to accommodate tubing lines of the fluid flow circuit 12. One of the pumps 20a may be provided as a source/recipient access pump, which may be associated with a source/recipient access line 22 of the fluid flow circuit 12 and operates to draw fluid from a fluid source (Fig. 5) and to return fluid to a fluid recipient (Figs. 6 and 7). Another one of the pumps 20b may be provided as an anticoagulant pump, which may be associated with an anticoagulant line 24 of the fluid flow circuit 12 and operates to add anticoagulant from an anticoagulant source or container 26 of the fluid flow circuit 12 (Fig. 5) to fluid drawn from the fluid source in the source/recipient access line 22 before the fluid enters into a blood separation module or chamber 28 of the fluid flow circuit 12. A third pump 20c may be provided as a concentrated fluid pump, which may be associated with a concentrated fluid outlet line 30 and operates to draw concentrated fluid from the blood separation chamber 28 and direct it into a concentrated fluid reservoir 32 after the fluid has been separated into a concentrated fluid and separated plasma in the blood separation chamber 28.

In the illustrated embodiment, the pumps 20a-20c are peristaltic pumps, but it is within the scope of the present disclosure for differently configured pumps, such as diaphragm or other pumps, to be provided. Furthermore, additional or alternative pumps may be provided without departing from the scope of the present disclosure. For example, a pump may be associated with a plasma outlet line 34 of the fluid flow circuit 12 to draw separated plasma from the blood separation chamber 28 after the plasma-containing fluid has been separated into a concentrated fluid and separated plasma. Also, as will be described in greater detail herein, the illustrated embodiment employs a single fluid flow tubing or flow path for both drawing blood from a source and flowing or returning fluids to a recipient, which are carried out intermittently. The system 10 could employ separate draw and return flow paths or tubes.

In addition to the pumps 20a-20c, the external components of the device 10 may include one or more clamps or valves 36a-36d (collectively referred to herein as a "clamp system") associated with the tubing lines of the fluid flow circuit 12. The clamps or valves 36a-36d may be variously configured and operate to selectively allow or prevent fluid flow through the associated tubing line. In the illustrated embodiment, one clamp or valve 36a may be provided as a fluid source/recipient clamp, which may be associated with a draw branch 22a of the source/recipient access line 22 of the fluid flow circuit 12 to allow (Fig. 5) or prevent (Figs. 6 and 7) the flow of fluid through the draw branch 22a of the source/recipient access line 22. Another one of the clamps or valves 36b may be provided as a reinfusion clamp or valve, which may be associated with a reinfusion branch 22b of the source/recipient access line 22 downstream of a concentrated fluid reservoir 32 of the fluid flow circuit 12 to allow (Figs. 6 and 7) or prevent (Fig. 5) the flow of concentrated fluid through the reinfusion branch 22b. A third clamp or valve 36c may be provided as a plasma clamp or valve, which may be associated with the plasma outlet line 34 to allow (Fig. 5) or prevent (Figs. 6 and 7) the flow of separated plasma through the plasma outlet line 34 and into a separated plasma container 38. A fourth clamp or valve 36d may be provided as a replacement fluid clamp or valve, which may be associated with a replacement fluid line 40 of the fluid flow circuit 12 to allow (Fig. 7) or prevent (Figs. 5 and 6) the flow of a replacement fluid out of a replacement fluid source 42 (e.g., a bag or container at least partially filled with saline). Additional or alternative clamps or valves may also be provided without departing from the scope of the present disclosure.

The illustrated device 10 further includes one or more pressure sensors 43a and 43b that may be associated with the fluid flow circuit 12 to monitor the pressure within one or more of the tubing lines of the fluid flow circuit 12 during operation of the pumps 20a-20c and clamps or valves 36a-36d. In one embodiment, one pressure sensor 43a may be associated with a tubing line that draws fluid from a fluid source and/or directs processed fluid to a fluid recipient, while the other pressure sensor 43b may be associated with a tubing line that directs fluid into or out of the blood separation chamber 28 to assess the pressure within the blood separation chamber 28, but the pressure sensors 43a and 43b may also be associated with other tubing lines without departing from the scope of the present disclosure. The pressure sensors 43a and 43b may send signals to the system controller 16 that are indicative of the pressure within the tubing line or lines being monitored by the pressure sensor 43a, 43b. If the controller 16 determines that an improper pressure is present within the fluid flow circuit 12 (e.g., a high pressure due to an occlusion of one of the tubing lines), then the controller 16 may instruct one or more of the pumps 20a-20c and/or one or more of the clamps or valves 36a-36d to act so as to alleviate the improper pressure condition (e.g., by reversing the direction of operation of one of the pumps 20a-20c and/or opening or closing one of the clamps or valves 36a-36d). Additional or alternative pressure sensors may also be provided without departing from the scope of the present disclosure.

The device 10 also includes a separation actuator 44 that interacts with a portion of the blood separation chamber 28 to operate the blood separation chamber 28. A chamber lock 46 may also be provided to hold the blood separation chamber 28 in place with respect to the cabinet 14 and in engagement with the separation actuator 44. The configuration and operation of the separation actuator 44 depends upon the configuration of the blood separation chamber 28. In the illustrated embodiment, the blood separation chamber 28 is provided as a spinning membrane-type separator, such as a separator of the type described in greater detail in U.S. Patents Nos. 5,194,145 and 5,234,608 or in PCT Patent Application Publication No. WO 2012/125457 A1. If provided as a spinning membrane-type separator, the blood separation chamber 28 may include a tubular housing 48 (Fig. 4), with a microporous membrane 50 positioned therein. An inlet 52 allows a fluid to enter into the housing 48 (via the draw branch 22a of the source/recipient access line 22), while a side outlet 54 allows concentrated fluid to exit the housing 48 (via the concentrated fluid outlet line 30) and a bottom outlet 56 allows separated plasma to exit the housing 48 (via the plasma outlet line 34) after the fluid has been separated into concentrated fluid and plasma.

In the illustrated embodiment, the separation actuator 44 is provided as a driver that is magnetically coupled to a rotor 58 on which the membrane 50 is mounted, with the separation actuator 44 causing the rotor 58 and membrane 50 to rotate about the central axis of the housing 48. The rotating rotor 58 and membrane 50 create Taylor vortices within a gap 60 between the housing 48 and the membrane 50, which tend to transport the concentrated fluid (which may be cellular blood components) away from the membrane 50 to exit the blood separation chamber 28 via the side outlet 54, while the separated plasma passes through the membrane 50 toward the central axis of the housing 48 to exit the blood separation chamber 28 via the bottom outlet 56. It should be understood that the illustrated and described blood separation chamber 28 is merely exemplary. For example, in other embodiments, a centrifugal device that separates fluid components based on density, rather than size, may be employed to separate a plasma-containing fluid into plasma and a concentrated fluid.

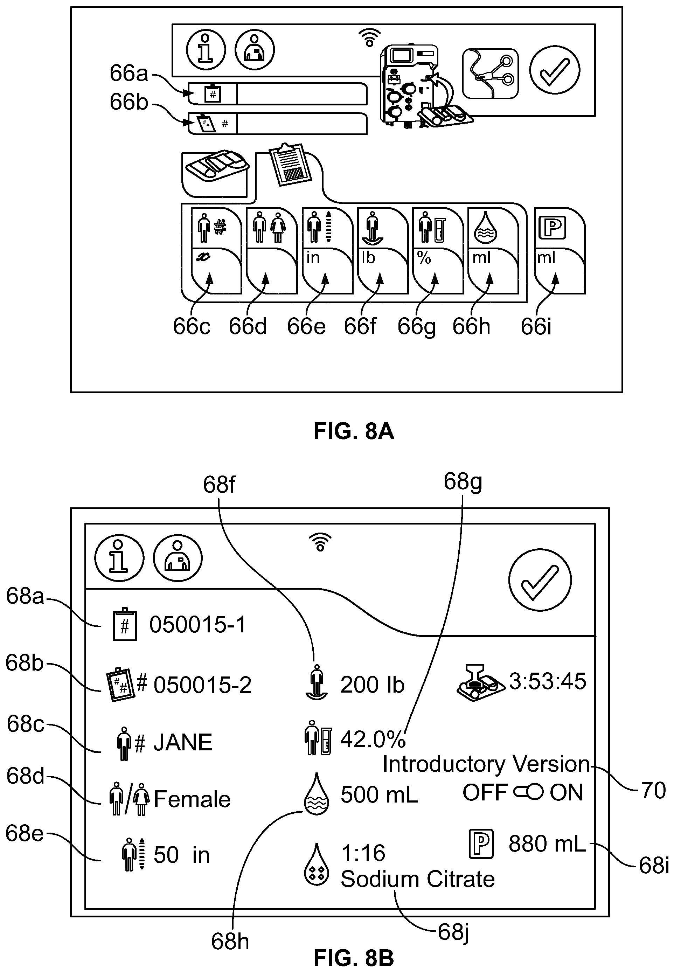

The device 10 may include alternative and/or additional components without departing from the scope of the present disclosure. For example, the illustrated device 10 includes a hemoglobin detector or optical sensor assembly 62 associated with the plasma outlet line 34, a blood cell weigh scale associated with the reservoir 32 and a plasma weight scale associated with the separated plasma container 38 of the fluid flow circuit 12. The illustrated device 10 also includes a data entry device 64 configured as a touch screen for inputting information into the controller 16 and displaying the input information or information originating from the controller 16. While a touch screen is illustrated (with Figs. 8A and 8B showing exemplary images that may be displayed on the touch screen), it should be understood that a differently configured data entry device (e.g., a barcode reader) may also be employed.

According to one method of using the illustrated blood separation system, a plasma-containing fluid is drawn from a fluid source into the blood separation chamber 28 during a draw phase or mode (Fig. 5), where it is separated into concentrated fluid and separated plasma. The separated plasma is retained by the device 10 (e.g., within the separated plasma container 38 of the fluid flow circuit 12), while the concentrated fluid is returned to a fluid recipient during a return or reinfusion phase or mode (Fig. 6). In one embodiment, the draw and return phases are repeatedly alternated (drawing from the fluid source, separating the fluid into plasma and concentrated fluid, and then returning the concentrated fluid to the fluid recipient) until a target (e.g., a collected amount of separated plasma) is achieved. All of the draw phases and all of the return phases may be identical or may differ from each other. For example, a final draw phase may draw less fluid from the fluid source than the previous draw phases and a final return phase may infuse a combination of concentrated fluid and replacement fluid to the fluid recipient, whereas the previous return phases return only concentrated fluid to the fluid recipient.

Fig. 7 shows an exemplary phase or mode in which replacement fluid (e.g., saline) is directed to the fluid recipient, either alone or with an amount of concentrated fluid. In the phase of Fig. 7, the clamp or valve 36d associated with the replacement fluid line 40 is opened to allow replacement fluid to flow out of the replacement fluid source 42. The clamp or valve 36a associated with the draw branch 22a of the source/recipient access line 22 may be in a closed condition to prevent fluid flow therethrough, such that the replacement fluid is directed into the blood separation chamber 28. The replacement fluid is pulled out of the blood separation chamber 28 and into the concentrated fluid reservoir 32 by operation of the pump 20c associated with the concentrated fluid outlet line 30. If there is any concentrated fluid in the concentrated fluid reservoir 32, then the replacement fluid mixes with the concentrated fluid prior to being pumped to the fluid recipient by the pump 20a associated with the source/recipient access line 22, otherwise the replacement fluid alone may be pumped to the fluid recipient. In one embodiment, the replacement fluid return mode of Fig. 7 is carried out only once, as a final return phase (e.g., when the amount of concentrated fluid in the concentrated fluid reservoir 32 is at a sufficiently low level) in which a mixture of concentrated fluid and replacement fluid is returned to the fluid recipient. This may be advantageous to ensure that all of the concentrated fluid in the concentrated fluid reservoir 32 (along with any remaining in the blood separation chamber 28) is rinsed out of the concentrated fluid reservoir 32 and pumped to the fluid recipient.

In other embodiments, the replacement fluid return mode of Fig. 7 may be carried out at other times, such as earlier in the procedure, at multiple scheduled times during a procedure, and/or at any time upon a request from the operator and/or using a different path between the replacement fluid source 42 and the fluid recipient. For example, it may be advantageous for the replacement fluid to bypass the blood separation chamber 28 and the concentrated fluid reservoir 32 if the replacement fluid is being pumped to a fluid recipient earlier in the procedure. In this case, the clamp or valve 36d associated with the replacement fluid line 40 and the clamp or valve 36a associated with the draw branch 22a of the source/recipient access line 22 may be opened to allow fluid flow therethrough, with the clamp or valve 36b associated with the reinfusion branch 22b in a closed condition to prevent fluid flow therethrough. The pump 20a associated with the source/recipient access line 22 may be activated (with the other two pumps 20b and 20c inactive) to draw replacement fluid out of the replacement fluid source 42 and through the replacement fluid line 40, the draw branch 22a, and finally the source/recipient access line 22 to the fluid recipient.

The illustrated fluid flow circuit 12 and procedure are a "single-needle" variation, with the same blood source access (e.g., a needle) being used to both draw blood from a blood source and return fluid to the blood source. In other embodiments, two blood source/recipient accesses are incorporated into the fluid flow circuit, which allows for a "double-needle" variation of the procedure in which blood may be drawn from a blood source (via a first needle or blood source access) at the same time that fluid is conveyed to the blood source or other recipient (via a second needle or recipient access). It should be understood that the present disclosure is not limited to either a single- or double-needle procedure or to any particular method of blood separation (e.g., via centrifugation or spinning membrane-type separator) or to any particular separation procedure (e.g., separation of blood into plasma and cellular blood components or separation of blood into plasma, buffy coat, and red blood cells).

Execution of a blood separation procedure by the controller 16 will depend upon data received by the controller 16, which is typically received from a data management system or entered by an operator using the data entry device 64. Figs. 8A and 8B show exemplary images that may be displayed on a screen of a data entry device 64, with Fig. 8A showing a pre-processing image that requests various information from the operator. The exemplary screen of Fig. 8A includes various fields 66a-66i for receiving and displaying data entered by the operator, which may include: a first field 66a for providing a procedure ID, a second field 66b for providing a donation setup ID, a third field 66c for providing a blood source ID, a fourth field 66d for providing a sex of a human blood source, a fifth field 66e for providing a height of a human blood source, a sixth field 66f for providing a weight of a human blood source, a seventh field 66g for providing information regarding the blood of the blood source (e.g., a hemoglobin or hematocrit level or platelet pre-count), an eighth field 66h for providing a procedure parameter (such as target saline volume), and a ninth field 66i for providing an additional procedure parameter (such as target plasma collection volume or a target combined volume of collected plasma and anticoagulant). It should be understood that the data requested in the screen of Fig. 8A is merely exemplary and that a data entry device 64 may request additional or different information from an operator.

The image of Fig. 8B may be considered as a summary or confirmation screen, which displays the various data entries after they have all been entered by the operator using the screen of Fig. 8A. In addition to nine fields 68a-68i that correspond to the nine fields of 66a-66i (respectively), the screen of Fig. 8B includes an additional field 68j that does not display data entered by the operator, but rather displays a center- or admin-configured value, which is an anticoagulant to whole blood ratio in the illustrated example. Field 68j may instead display a calculated value derived from one or more of the entries provided by the operator using the data entry device 64, or the screen may be provided with one or more additional fields each presenting a different calculated value. This calculated value may be any of a number of possible values including, without limitation, an anticoagulant to whole blood ratio, body mass index of a human source (which may be calculated using the height and weight of the blood source), the total blood volume of the source (which may be estimated using the height and weight of a human blood source according to a known approach, such as use of the Lemmens-Bernstein-Brodsky equation, or a novel approach), the total red blood cell or plasma volume of the source, the extracellular fluid volume of the source, and the estimated time required to complete the procedure. It is also possible to have a summary or confirmation screen to display a plurality of calculated values and/or for multiple summary or confirmation screens (each displaying one or more calculated values) to be presented.

The controller 16 receives the data from the data management system or entered by the operator using the data entry device 64, along with calculating or being provided with the calculated values. The controller 16 then controls the other components of the device 10 to execute a selected blood separation procedure.

According to an aspect of the present disclosure, the controller 16 is configured to implement two different versions of the same blood separation procedure, which versions are referred to herein as an "introductory" or "gentle" version and a "standard" version. The "introductory" version of a given procedure is intended to be implemented for a blood source that may not be suited for the "standard" version for any of a number of reasons, with the "introductory" version being intended to improve donor tolerance of the procedure by reducing minor/moderate reactions. According to another aspect of the present disclosure, it is possible for a blood source to "graduate" from the "introductory" version of a procedure, by successfully undergoing the "introductory" version of the procedure one or more times in order to establish that they are ready for the "standard" version. Once such a blood source has established that they are ready for the "standard" version of the procedure, that version may be implemented for that blood source for future iterations of the procedure.

The "introductory" version of a blood separation procedure may differ from the "standard" version in any of a number of ways. For example, the separation efficiency of the blood separation device 10 may be greater in the "standard" version than in the "introductory" version. In the above-described procedure (in which cellular blood components are separated from plasma), this difference would take the form of a relatively high blood cell separation efficiency when implementing the "standard" version of the procedure and a lower blood cell separation efficiency when implementing the "introductory" version of the procedure.

According to another possible difference, a greater percentage of the blood and/or red blood cells of the blood source may be present in the fluid flow circuit 12 at any particular time during implementation of the "standard" version of a procedure than during implementation of the "introductory" version. For example, as described above, it is possible to calculate the total blood volume or pre-procedure red blood cell volume of a blood source. When implementing the "introductory" version of a procedure, the controller 16 may actuate the other components of the blood separation device 10 to allow for no more than 14% of the calculated total blood volume and/or pre-procedure red blood cell volume of the blood source to be present in the fluid flow circuit 12 at any particular time. When implementing the "standard" version of the same procedure, the controller 16 may actuate the other components of the blood separation device 10 to allow for a greater percentage of the calculated total blood volume and/or pre-procedure red blood cell volume of the blood source (e.g., up to 16%) to be present in the fluid flow circuit 12 at any particular time. Alternatively, different limits may be placed on the volume of blood or red blood cells of a blood source that may be present in the fluid flow circuit 12 at any particular time during implementation of a procedure, with there being a greater limit (e.g., 250 ml of red blood cells or 600 ml of blood) for a "standard" version of a procedure than for an "introductory" version (e.g., 200 ml of red blood cells or 500 ml of blood).

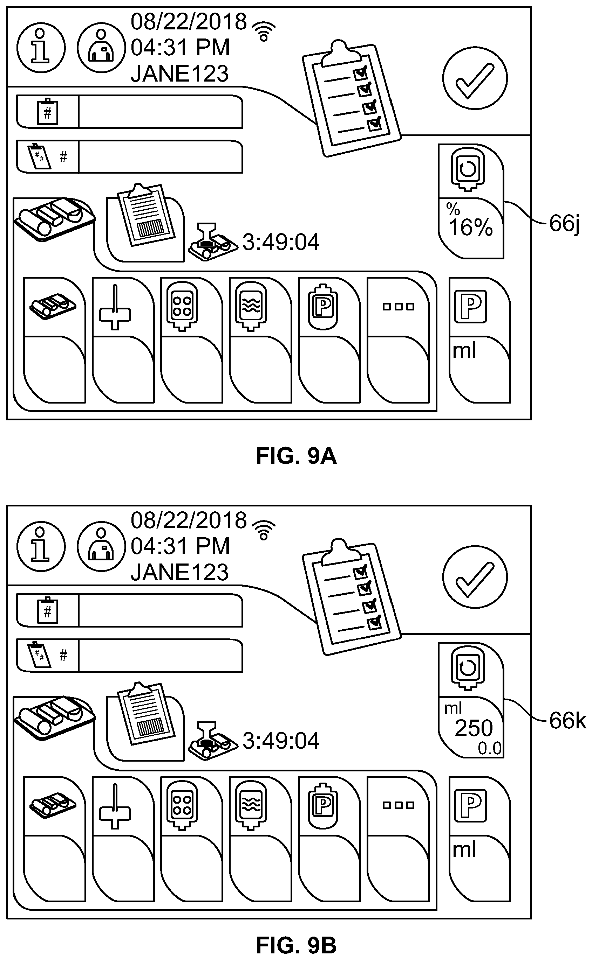

It should be understood that 14% and 16% are merely exemplary and that other percentages may be employed as limits for the "introductory" and "standard" versions of a procedure. According to one aspect of the present disclosure, a blood draw center or facility may be enabled to set a range of percentages that may be used during a procedure. For example, a blood draw center may set a range of 12-16% of the calculated total blood and/or pre-procedure red blood cell volume of a blood source for the maximum allowable volume of blood and/or red blood cells that may be present in a fluid flow circuit 12 at any particular time during a procedure. An operator may then instruct the controller 16 (e.g., using the data entry device 64) which percentage within the predetermined range to employ when executing one of the versions of a blood separation procedure, as shown in Fig. 9A at icon 66j.

Similarly, it should be understood that 200 ml and 250 ml of red blood cells and 500 ml and 600 ml of blood are merely exemplary and that other volumes may be employed as limits for the "introductory" and "standard" versions of a procedure. According to one aspect of the present disclosure, a blood draw center or facility may be enabled to set a range of maximum volumes that may be used during a procedure. For example, a blood draw center may set a range of 150-250 ml for the maximum allowable volume of red blood cells and/or 450-600 ml for the maximum allowable volume of blood that may be present in a fluid flow circuit 12 at any particular time during a procedure. An operator may then instruct the controller 16 (e.g., using the data entry device 64) which volume within the predetermined range to employ when executing one of the versions of a blood separation procedure, as shown in Fig. 9B at icon 66k.

According to yet another possible difference, a relatively large blood separation chamber 28 (allowing for a greater extracorporeal fluid volume) may be employed when implementing the "standard" version of a blood separation procedure, with a relatively small blood separation chamber 28 (allowing for a smaller extracorporeal fluid volume) may be employed when implementing the "introductory" version of the same procedure. The particular size and configuration of such "standard" and "introductory" blood separation chambers may vary without departing from the scope of the present disclosure. However, in an exemplary embodiment, the sizes of the "standard" and "introductory" blood separation chambers may be selected based on the allowable extracorporeal fluid volume for the associated version of the procedure (e.g., with an "introductory" blood separation chamber being configured such that no more than 14% of the blood of a blood source can be present in the fluid flow circuit 12 at any one time when implementing the "introductory" version of a procedure and with a "standard" blood separation chamber being configured such that up to 16% of the blood of a blood source can be present in the fluid flow circuit 12 at any one time during a "standard" version of that same procedure).

According to another possible difference, one or more stages of a procedure may be enhanced when implementing the "standard" version of a blood separation procedure. For example, the blood draw stage (as shown in Fig. 5) executed during a "standard" version of a blood separation procedure may have a different duration than the same stage during an "introductory" version of the same procedure. The controller 16 may also (or alternatively) actuate the pump system to draw blood from the blood source at a different (typically higher) rate during a "standard" version of the blood draw stage compared to the blood draw rate during an "introductory" version. This may include different minimum and/or maximum draw rates in each version of the procedure. In general, more blood may be drawn from the blood source during the blood draw stage of a "standard" version of a procedure than during the blood draw stage of an "introductory" version of the same procedure. In embodiments in which multiple blood draw stages are executed (typically "single-needle" procedures), it may be advantageous for the first blood draw stage of an "introductory" version of a procedure to have a particularly low draw rate (for donor comfort), with the draw rate increasing in one or more subsequent blood draw stages.

Similarly, a fluid return stage (as shown in Figs. 6 and 7) executed during a "standard" version of a blood separation procedure may have a different duration than the same stage during an "introductory" version of the same procedure. The controller 16 may also (or alternatively) actuate the pump system to convey fluid to a recipient (e.g., the blood source) at a different rate during a "standard" version of the fluid return stage compared to the fluid return rate during an "introductory" version. This may include different minimum and/or maximum return rates in each version of the procedure. In general, more fluid may be conveyed to the recipient during the fluid return stage of a "standard" version of a procedure than during the fluid return stage of an "introductory" version of the same procedure (as more blood may be drawn from the source during the "standard" version). In embodiments in which multiple fluid returns stages are executed (typically "single-needle" procedures), it may be advantageous for the first fluid return stage of an "introductory" version of a procedure to have a particularly low return rate (for donor comfort), with the return rate increasing in one or more subsequent fluid return stages.

According to one aspect of the present disclosure, the parameters of one or both versions of the procedure may be configured upon the device 10 being installed and set up and/or may be adjusted by an operator before or at the beginning of a procedure. This may include the ranges or limits on the parameters that may be selected by an operator (e.g., if an operator is limited to selecting a maximum extracorporeal blood volume from a range of values).

The criteria by which a blood source is designated for the "introductory" version of a blood separation procedure or the "standard" version may vary without departing from the scope of the present disclosure. This screening process may involve a blood source being designated as qualified for the "introductory" version, qualified for the "standard" version, not qualified for the "introductory" version, or not qualified for the "standard" version. By way of example, the age of a blood source may be a factor in determining which version of a blood separation procedure to implement, with the "introductory" version tending to be more suitable for being especially young and especially old blood sources. An additional or alternative factor may be the weight of a blood source, with the "introductory" version tending to be more suitable for lower weight blood sources. The sex of a blood source may be another factor (which may be considered together with other factors or independently), with the "introductory" version more frequently being suitable for female blood sources. Yet another factor may be the day-of-condition of the blood source (e.g., if there is anything about the status of the blood source on the day of a procedure which suggests that a more gentle version may be advisable).

Yet another consideration is whether the blood separation procedure (or a comparable procedure) was previously implemented for the blood source. If the blood source has never undergone the procedure (or a comparable procedure), the "introductory" version of the procedure may be more suitable than the "standard" version. If the blood source has previously undergone the procedure (or a comparable procedure), it may be advantageous to consider the number of times that blood has been drawn from the blood source, which may include the number of times that blood has been drawn from the blood source in a period of time (e.g., to assess whether the blood source is an active and regular participant or if their participation has recently lapsed) and/or the average frequency or number of times that blood has been drawn from the blood source over the relevant time period.

The blood draw history of the blood source may be implemented as a factor in any of a number of ways. In one embodiment, the "history" factor may be treated as a status, with a blood source being categorized as "new" or "lapsed" or "regular," for example. A blood source who has never undergone a particular procedure (or a comparable procedure) may be categorized as a "new" blood source. A blood source who has previously undergone a particular procedure (or a comparable procedure) in the past, but not sufficiently recently (or not recently on a sufficiently regular basis) may be categorized as a "lapsed" blood source. A blood source who has recently and regularly undergone a particular procedure (or a comparable procedure) may be categorized as a "regular" blood source. The different statuses may be given different numerical values or "points" by the controller 16 (e.g., 0 for a "new" blood source, 1 for a "lapsed" blood source, and 2 for a "regular" blood source) when determining which version of a blood separation procedure to implement, with a blood source having a "regular" status being more likely than a "lapsed" or "new" blood source to be considered suitable for implementation of the "standard" version (on account of having more "points").

In another embodiment, the "history" factor may be treated as a number equal to the number of times that blood has been previously drawn from the blood source or the number of times that the blood source has undergone a particular procedure (or a comparable procedure). If different types of procedures are included in the count, certain procedures (e.g., ones that are the same as the particular procedure to be implemented) may be given a greater numerical value or weight than other procedures (e.g., ones that are similar to, but different from, the procedure to be implemented). Similar to the embodiment in which the "history" factor is treated as a status, a blood source who has more frequently undergone a particular procedure (or comparable procedures) may be more likely to qualify for the "standard" version of a blood separation procedure than a less experienced blood source on account of having more "points" or progress towards a qualifying threshold value.

If multiple factors (e.g., the age, sex, and blood draw history of a blood source) are considered when determining which version of a procedure to implement, they may be given the same or different weights. For example, it may be the case that the blood draw history of a blood source may be more relevant (and thus be entitled to more weight) than the age and sex of the blood source. If a blood source is of an age and sex typically suited for the "introductory" version of a procedure, those factors may be outweighed by the blood source having a history of successfully undergoing the "standard" version of the procedure (or having successfully undergone similar blood separation procedures).

As some of the above factors may change during the lifetime of a blood source, they may be reassessed periodically to determine whether a subsequent blood separation procedure should be executed according to the "introductory" version or the "standard" version. Indeed, while it may be more typical for a blood source to "graduate" from the "introductory" version of a procedure to the "standard" version (e.g., by undergoing the "introductory" version of the procedure a certain number of times), it is also possible for a blood source to be transitioned from the "standard" version to the "introductory" version (e.g., if the blood source undergoes a significant weight loss).

The determination as to which version of a blood separation procedure to implement (including whether to change a blood source from one version to the other) may be made in any of a number of ways. For example, based on information provided to it (e.g., by an operator via the data entry device 64 or via a data management system), the controller 16 may determine which version of a procedure to implement and then proceed to implement that version of the procedure without input from an operator. In another embodiment, the controller 16 may provide the operator with a recommended version of the procedure, with the operator having the option of either implementing the recommended version or a different version. This may include the operator being provided with an icon 70 (Fig. 8B) or button to manipulate to initiate a selected version of a procedure, with the appropriate parameters (e.g., draw and return rates, volume of blood to be processed each cycle, maximum allowable extracorporeal blood volume, maximum allowable extracorporeal red blood cell volume, etc.) being automatically implemented by the controller 16. In yet another embodiment, the operator may be enabled to select (e.g., using an icon 70 or button or the like) the version of the procedure to implement without receiving input from the controller 16. In still another embodiment, some other authority (e.g., a "front desk" operator or central controller) may be enabled to make the determination of which version of a procedure to implement. Instructing the controller 16 to execute one version of a procedure or another may be as simple as setting a single flag value in a procedure setup file or may be implemented in any other way.

While "introductory" and "standard" versions of a blood separation procedure are described herein, it should be understood that the present disclosure is not limited to execution of an "introductory" or "gentle" version of a procedure and a "standard" version of the same procedure. For example, two "standard" versions of a blood separation procedure may available, with the two versions being substantially equivalent alternatives that differ in some way, rather than one version being presented as a "gentle" version of the procedure. Indeed, the manner in which two versions of a particular blood separation procedure differ may vary.

While implementation of "introductory" and "standard" versions of a given blood separation procedure is described above as being executed by a single blood separation device 10, it should be understood that multiple blood separation devices (which may be similarly or differently configured) may instead be employed. According to this approach, a first device may be configured to execute a first version of the procedure (e.g., the "introductory" version), with a second device being configured to execute the second version of the procedure (e.g., the "standard" version). Thus, a blood source may "graduate" from the first or "introductory" version of the procedure to the second or "standard" version by transitioning from use of the first device to use of the second device.

It will be understood that the embodiments and examples described above are illustrative of some of the applications of the principles of the present subject matter. Numerous modifications may be made by those skilled in the art. For these reasons, the scope is as set forth in the following claims.

## Claims

1. A blood separation device (10), comprising:
a pump system (20a-20c);
a clamp system (36a-36d);
a separation actuator (44); and
a controller (16) programmed with a plurality of procedural parameters of a first version and a second version of one blood separation procedure in which the pump system (20a-20c), the clamp system (36a-36d), and the separation actuator (44) are configured to be actuated to draw blood from a blood source, separate the blood into at least two blood components, and convey at least a portion of one of said blood components to a recipient, wherein at least one of the plurality of procedural parameters of the first version is different from the corresponding procedural parameter of the second version,
wherein the controller (16) is configured to
determine or be instructed that the blood source is qualified for the first version or the second version and/or determine or be instructed that the blood source is not qualified for the first version or the second version,
implement the blood separation procedure in the first version for the blood source upon determining or being instructed that the blood source is qualified for the first version and/or upon determining or being instructed that the blood source is not qualified for the second version, and
implement the blood separation procedure in the second version for the blood source upon determining or being instructed that the blood source is qualified for the second version and/or upon determining or being instructed that the blood source is not qualified for the first version, wherein the blood separation procedure includes the same phases when being implemented in the first version and in the second version.

2. The blood separation device (10) of claim 1, wherein implementation of the blood separation procedure in the first version vs. the second version is based at least in part on an age and/or a weight and/or a sex of the blood source and/or whether the blood separation procedure was previously implemented for the blood source and/or the number of times that blood has been drawn from the blood source.

3. The blood separation device (10) of claim 2, wherein assessing whether the blood separation procedure was previously implemented for the blood source includes
categorizing the blood source as a "new" blood source when the blood separation procedure was never previously implemented for the blood source,
categorizing the blood source as a "lapsed" blood source when a predetermined amount of time has elapsed since the blood separation procedure was last implemented for the blood source, and
categorizing the blood source as a "regular" blood source when the blood separation procedure has been implemented for the blood source more recently than a predetermined amount of time.

4. The blood separation device (10) of any one of claims 2-3, wherein
assessing whether the blood separation procedure was previously implemented for the blood source includes assigning one of a plurality of statuses to the blood source based at least in part on whether the blood separation procedure was previously implemented for the blood source, and
each of the plurality of statuses is given a different value or weight when determining whether the blood source is qualified for the first version or the second version and/or determining whether the blood source is not qualified for the first version or the second version.

5. The blood separation device (10) of any one of claims 2-4, wherein said number of times that blood has been drawn from the blood source includes the number of times that blood has been drawn from the blood source in a period of time or the average number of times that blood has been drawn from the blood source in the period of time.

6. The blood separation device (10) of any one of claims 2-5, wherein assessing the number of times that blood has been drawn from the blood source includes determining the type of a previous blood draw procedure implemented for the blood source during which blood was drawn from the blood source, preferably by selecting from a plurality of types of blood draw procedures and giving different values or weights to at least two different types of blood draw procedures when determining whether the blood source is qualified for the first version or the second version and/or determining whether a blood source is not qualified for the first version or the second version.

7. The blood separation device (10) of claim 6, wherein
a first one of said types of blood draw procedures is the blood separation procedure to be implemented for the blood source,
a second one of said types of blood draw procedures is not the blood separation procedure to be implemented for the blood source, and
a greater value or weight is given to said first type of blood draw procedure than to said second type of blood draw procedure when determining whether the blood source is qualified for the first version or the second version and/or determining whether a blood source is not qualified for the first version or the second version.

8. The blood separation device (10) of any one of claims 1-7, wherein
the blood separation procedure includes separating blood from the blood source into cellular blood components and plasma, and
a blood cell separation efficiency of the blood separation procedure in the first version is different from a blood cell separation efficiency of the blood separation procedure in the second version.

9. The blood separation device (10) of any one of claims 1-8, wherein
the blood separation procedure includes separating blood from the blood source into cellular blood components and plasma in a fluid flow circuit (12),
the controller is further configured to determine or be provided with a pre-procedure red blood cell volume of the blood source,
a volume of red blood cells less than a selected percentage of the pre-procedure red blood cell volume is allowed to be present in the fluid flow circuit (12) at any time when implementing the blood separation procedure in the first version, and
a volume of red blood cells different from said selected percentage of the pre-procedure red blood cell volume is allowed to be present in the fluid flow circuit (12) at at least one time when implementing the blood separation procedure in the second version.

10. The blood separation device (10) of any one of claims 1-8, wherein
the blood separation procedure includes separating blood from the blood source into cellular blood components and plasma in a fluid flow circuit (12),
the controller is further configured to determine or be provided with a total blood volume of the blood source,
a volume of blood less than a selected percentage of the total blood volume is allowed to be present in the fluid flow circuit (12) at any time when implementing the blood separation procedure in the first version, and
a volume of blood different from said selected percentage of the total blood volume is allowed to be present in the fluid flow circuit (12) at at least one time when implementing the blood separation procedure in the second version.

11. The blood separation device (10) of any one of claims 1-10, wherein
implementing the blood separation procedure in the first version includes separating blood from the blood source in a first blood separation chamber (28), and
implementing the blood separation procedure in the second version includes separating blood from the blood source in a second blood separation chamber (28) having a different volume than the first blood separation chamber (28).

12. The blood separation device (10) of any one of claims 1-11, wherein
implementing the blood separation procedure in the first version includes executing a first blood draw phase having a duration, a draw rate, and a blood draw volume, and
implementing the blood separation procedure in the second version includes executing a second blood draw phase having a different duration, draw rate, and/or blood draw volume than said first blood draw phase.

13. The blood separation device (10) of any one of claims 1-12, wherein
implementing the blood separation procedure in the first version includes executing a first fluid return phase having a duration, a return rate, and a fluid return volume, and
implementing the blood separation procedure in the second version includes executing a second fluid return phase having a different duration, return rate, and/or fluid return volume than said first fluid return phase.

14. The blood separation device (10) of any one of claims 1-13, further comprising a data entry device (64) including a button or icon (70) configured to be manipulated by an operator to instruct the controller (16) to implement the blood separation procedure in the first version.

15. The blood separation device (10) of any one of claims 1-13, further comprising a data entry device (64) configured to receive entered data regarding a blood source, wherein the controller (16) is configured to determine whether to implement the blood separation procedure in the first version vs. the second version based at least in part on said entered data.

## Patentansprüche

1. Bluttrennvorrichtung (10), umfassend:
ein Pumpensystem (20a-20c);
ein Klemmsystem (36a-36d);
einen Trennaktor (44); und
eine Steuerung (16), die mit einer Mehrzahl von Verfahrensparametern einer ersten Version und einer zweiten Version eines Bluttrennverfahrens programmiert ist, bei dem das Pumpensystem (20a-20c), das Klemmsystem (36a-36d) und der Trennaktor (44) dazu konfiguriert sind, betätigt zu werden, um Blut aus einer Blutquelle zu entnehmen, das Blut in mindestens zwei Blutkomponenten zu trennen und mindestens einen Teil einer der genannten Blutkomponenten zu einem Empfänger zu befördern, wobei mindestens einer der Mehrzahl von Verfahrensparametern der ersten Version von dem entsprechenden Verfahrensparameter der zweiten Version verschieden ist,
wobei die Steuerung (16) dazu konfiguriert ist,
zu bestimmen oder angewiesen zu werden, dass die Blutquelle für die erste Version oder die zweite Version qualifiziert ist, und/oder zu bestimmen oder angewiesen zu werden, dass die Blutquelle für die erste Version oder die zweite Version nicht qualifiziert ist,
das Bluttrennverfahren in der ersten Version für die Blutquelle auszuführen, wenn bestimmt oder angewiesen wird, dass die Blutquelle für die erste Version qualifiziert ist, und/oder wenn bestimmt oder angewiesen wird, dass die Blutquelle für die zweite Version nicht qualifiziert ist, und
das Bluttrennverfahren in der zweiten Version für die Blutquelle auszuführen, wenn bestimmt oder angewiesen wird, dass die Blutquelle für die zweite Version qualifiziert ist, und/oder wenn bestimmt oder angewiesen wird, dass die Blutquelle für die erste Version nicht qualifiziert ist, wobei das Bluttrennverfahren dieselben Phasen umfasst, wenn es in der ersten Version und in der zweiten Version ausgeführt wird.

2. Bluttrennvorrichtung (10) nach Anspruch 1, wobei die Ausführung des Bluttrennverfahrens in der ersten Version gegenüber der zweiten Version zumindest teilweise auf einem Alter und/oder einem Gewicht und/oder einem Geschlecht der Blutquelle und/oder darauf beruht,
ob das Bluttrennverfahren zuvor für die Blutquelle ausgeführt wurde und/oder wie oft Blut aus der Blutquelle entnommen wurde.

3. Bluttrennvorrichtung (10) nach Anspruch 2, wobei das Beurteilen, ob das Bluttrennverfahren zuvor für die Blutquelle ausgeführt wurde, umfasst:
Kategorisieren der Blutquelle als eine "neue" Blutquelle, wenn das Bluttrennverfahren niemals zuvor für die Blutquelle ausgeführt wurde,
Kategorisieren der Blutquelle als eine "ausgesetzte" Blutquelle, wenn eine vorbestimmte Zeitspanne verstrichen ist, seitdem das Bluttrennverfahren zuletzt für die Blutquelle ausgeführt wurde, und
Kategorisieren der Blutquelle als eine "reguläre" Blutquelle, wenn das Bluttrennverfahren für die Blutquelle vor kürzerer Zeit als einer vorbestimmten Zeitspanne ausgeführt wurde.

4. Bluttrennvorrichtung (10) nach einem der Ansprüche 2 bis 3, wobei
das Beurteilen, ob das Bluttrennverfahren zuvor für die Blutquelle ausgeführt wurde, umfasst, der Blutquelle einen von einer Mehrzahl von Status zuzuweisen, zumindest teilweise basierend darauf, ob das Bluttrennverfahren zuvor für die Blutquelle ausgeführt wurde, und
jedem der Mehrzahl von Status ein unterschiedlicher Wert oder ein unterschiedliches Gewicht zugeordnet wird, wenn bestimmt wird, ob die Blutquelle für die erste Version oder die zweite Version qualifiziert ist, und/oder wenn bestimmt wird, ob die Blutquelle für die erste Version oder die zweite Version nicht qualifiziert ist.

5. Bluttrennvorrichtung (10) nach einem der Ansprüche 2 bis 4, wobei die genannte Anzahl der Male, die Blut aus der Blutquelle entnommen wurde, die Anzahl der Male umfasst, die Blut aus der Blutquelle in einer Zeitspanne entnommen wurde, oder die durchschnittliche Anzahl der Male, die Blut aus der Blutquelle in der Zeitspanne entnommen wurde.

6. Bluttrennvorrichtung (10) nach einem der Ansprüche 2 bis 5, wobei das Beurteilen der Anzahl der Male, die Blut aus der Blutquelle entnommen wurde, das Bestimmen der Art eines vorherigen Blutentnahmeverfahrens umfasst, das für die Blutquelle ausgeführt wurde und während dessen Blut aus der Blutquelle entnommen wurde, vorzugsweise durch Auswählen aus einer Mehrzahl von Arten von Blutentnahmeverfahren und Zuordnen unterschiedlicher Werte oder Gewichte zu mindestens zwei unterschiedlichen Arten von Blutentnahmeverfahren, wenn bestimmt wird, ob die Blutquelle für die erste Version oder die zweite Version qualifiziert ist, und/oder wenn bestimmt wird, ob eine Blutquelle für die erste Version oder die zweite Version nicht qualifiziert ist.

7. Bluttrennvorrichtung (10) nach Anspruch 6, wobei
eine erste der genannten Arten von Blutentnahmeverfahren das Bluttrennverfahren ist, das für die Blutquelle auszuführen ist,
eine zweite der genannten Arten von Blutentnahmeverfahren nicht das Bluttrennverfahren ist, das für die Blutquelle auszuführen ist, und
der genannten ersten Art von Blutentnahmeverfahren ein größerer Wert oder ein größeres Gewicht zugeordnet wird als der genannten zweiten Art von Blutentnahmeverfahren, wenn bestimmt wird, ob die Blutquelle für die erste Version oder die zweite Version qualifiziert ist, und/oder
wenn bestimmt wird, ob eine Blutquelle für die erste Version oder die zweite Version nicht qualifiziert ist.

8. Bluttrennvorrichtung (10) nach einem der Ansprüche 1 bis 7, wobei
das Bluttrennverfahren das Trennen von Blut aus der Blutquelle in zelluläre Blutkomponenten und Plasma umfasst, und
eine Blutzell-Trenneffizienz des Bluttrennverfahrens in der ersten Version von einer Blutzell-Trenneffizienz des Bluttrennverfahrens in der zweiten Version verschieden ist.

9. Bluttrennvorrichtung (10) nach einem der Ansprüche 1 bis 8, wobei
das Bluttrennverfahren das Trennen von Blut aus der Blutquelle in zelluläre Blutkomponenten und Plasma in einem Fluidströmungskreislauf (12) umfasst,
die Steuerung ferner dazu konfiguriert ist, ein vor dem Verfahren vorliegendes Volumen roter Blutkörperchen der Blutquelle zu bestimmen oder mit diesem versehen zu werden,
zugelassen ist, dass ein Volumen roter Blutkörperchen, das kleiner ist als ein ausgewählter Prozentsatz des vor dem Verfahren vorliegenden Volumens roter Blutkörperchen, zu jedem Zeitpunkt beim Ausführen des Bluttrennverfahrens in der ersten Version in dem Fluidströmungskreislauf (12) vorhanden ist, und
zugelassen ist, dass ein Volumen roter Blutkörperchen, das von dem genannten ausgewählten Prozentsatz des vor dem Verfahren vorliegenden Volumens roter Blutkörperchen verschieden ist, zu mindestens einem Zeitpunkt beim Ausführen des Bluttrennverfahrens in der zweiten Version in dem Fluidströmungskreislauf (12) vorhanden ist.

10. Bluttrennvorrichtung (10) nach einem der Ansprüche 1 bis 8, wobei
das Bluttrennverfahren das Trennen von Blut aus der Blutquelle in zelluläre Blutkomponenten und Plasma in einem Fluidströmungskreislauf (12) umfasst,
die Steuerung ferner dazu konfiguriert ist, ein Gesamtblutvolumen der Blutquelle zu bestimmen oder mit diesem versehen zu werden,
zugelassen ist, dass ein Blutvolumen, das kleiner ist als ein ausgewählter Prozentsatz des Gesamtblutvolumens, zu jedem Zeitpunkt beim Ausführen des Bluttrennverfahrens in der ersten Version in dem Fluidströmungskreislauf (12) vorhanden ist, und
zugelassen ist, dass ein Blutvolumen, das von dem genannten ausgewählten Prozentsatz des Gesamtblutvolumens verschieden ist, zu mindestens einem Zeitpunkt beim Ausführen des Bluttrennverfahrens in der zweiten Version in dem Fluidströmungskreislauf (12) vorhanden ist.

11. Bluttrennvorrichtung (10) nach einem der Ansprüche 1 bis 10, wobei
das Ausführen des Bluttrennverfahrens in der ersten Version das Trennen von Blut aus der Blutquelle in einer ersten Bluttrennkammer (28) umfasst, und
das Ausführen des Bluttrennverfahrens in der zweiten Version das Trennen von Blut aus der Blutquelle in einer zweiten Bluttrennkammer (28) umfasst, die ein anderes Volumen als die erste Bluttrennkammer (28) aufweist.

12. Bluttrennvorrichtung (10) nach einem der Ansprüche 1 bis 11, wobei
das Ausführen des Bluttrennverfahrens in der ersten Version das Ausführen einer ersten Blutentnahmephase mit einer Dauer, einer Entnahmerate und einem Blutentnahmevolumen umfasst, und
das Ausführen des Bluttrennverfahrens in der zweiten Version das Ausführen einer zweiten Blutentnahmephase umfasst, die eine andere Dauer, Entnahmerate und/oder ein anderes Blutentnahmevolumen als die genannte erste Blutentnahmephase aufweist.

13. Bluttrennvorrichtung (10) nach einem der Ansprüche 1 bis 12, wobei
das Ausführen des Bluttrennverfahrens in der ersten Version das Ausführen einer ersten Fluidrückführphase mit einer Dauer, einer Rückführrate und einem Fluidrückführvolumen umfasst, und
das Ausführen des Bluttrennverfahrens in der zweiten Version das Ausführen einer zweiten Fluidrückführphase umfasst, die eine andere Dauer, Rückführrate und/oder ein anderes Fluidrückführvolumen als die genannte erste Fluidrückführphase aufweist.

14. Bluttrennvorrichtung (10) nach einem der Ansprüche 1 bis 13, ferner umfassend eine Dateneingabevorrichtung (64), die eine Schaltfläche oder ein Symbol (70) umfasst, die bzw. das dazu konfiguriert ist, von einem Bediener betätigt zu werden, um die Steuerung (16) anzuweisen, das Bluttrennverfahren in der ersten Version auszuführen.

15. Bluttrennvorrichtung (10) nach einem der Ansprüche 1 bis 13, ferner umfassend eine Dateneingabevorrichtung (64), die dazu konfiguriert ist, eingegebene Daten bezüglich einer Blutquelle zu empfangen, wobei die Steuerung (16) dazu konfiguriert ist, zumindest teilweise basierend auf den genannten eingegebenen Daten zu bestimmen, ob das Bluttrennverfahren in der ersten Version gegenüber der zweiten Version auszuführen ist.

## Revendications

1. Dispositif de séparation sanguine (10), comprenant :
un système de pompage (20a-20c) ;
un système de pinçage (36a-36d) ;
un actionneur de séparation (44) ; et
un contrôleur (16) programmé avec une pluralité de paramètres procéduraux d'une première version et d'une seconde version d'une même procédure de séparation sanguine dans laquelle le système de pompage (20a-20c), le système de pinçage (36a-36d) et l'actionneur de séparation (44) sont configurés pour être actionnés afin de prélever du sang d'une source sanguine, séparer le sang en au moins deux composants sanguins, et acheminer au moins une partie de l'un desdits composants sanguins vers un receveur, dans lequel au moins l'un de la pluralité de paramètres procéduraux de la première version est différent du paramètre procédural correspondant de la seconde version,
dans lequel le contrôleur (16) est configuré pour
déterminer ou recevoir l'instruction que la source sanguine est qualifiée pour la première version ou la seconde version et/ou déterminer ou recevoir l'instruction que la source sanguine n'est pas qualifiée pour la première version ou la seconde version,
mettre en œuvre la procédure de séparation sanguine dans la première version pour la source sanguine lorsqu'il est déterminé ou qu'il est reçu l'instruction que la source sanguine est qualifiée pour la première version et/ou lorsqu'il est déterminé ou qu'il est reçu l'instruction que la source sanguine n'est pas qualifiée pour la seconde version, et
mettre en œuvre la procédure de séparation sanguine dans la seconde version pour la source sanguine lorsqu'il est déterminé ou qu'il est reçu l'instruction que la source sanguine est qualifiée pour la seconde version et/ou lorsqu'il est déterminé ou qu'il est reçu l'instruction que la source sanguine n'est pas qualifiée pour la première version, dans lequel la procédure de séparation sanguine comprend les mêmes phases lorsqu'elle est mise en œuvre dans la première version et dans la seconde version.

2. Dispositif de séparation sanguine (10) selon la revendication 1, dans lequel la mise en œuvre de la procédure de séparation sanguine dans la première version par rapport à la seconde version est fondée au moins en partie sur un âge et/ou un poids et/ou un sexe de la source sanguine et/ou sur le fait que
la procédure de séparation sanguine a été précédemment mise en œuvre pour la source sanguine et/ou le nombre de fois que du sang a été prélevé de la source sanguine.

3. Dispositif de séparation sanguine (10) selon la revendication 2, dans lequel l'évaluation de la question de savoir si la procédure de séparation sanguine a été précédemment mise en œuvre pour la source sanguine comprend :
la catégorisation de la source sanguine comme source sanguine « nouvelle » lorsque la procédure de séparation sanguine n'a jamais été précédemment mise en œuvre pour la source sanguine,
la catégorisation de la source sanguine comme source sanguine « inactive » lorsqu'une quantité prédéterminée de temps s'est écoulée depuis que la procédure de séparation sanguine a été mise en œuvre pour la dernière fois pour la source sanguine, et
la catégorisation de la source sanguine comme source sanguine « régulière » lorsque la procédure de séparation sanguine a été mise en œuvre pour la source sanguine plus récemment qu'une quantité prédéterminée de temps.

4. Dispositif de séparation sanguine (10) selon l'une quelconque des revendications 2 à 3, dans lequel
l'évaluation de la question de savoir si la procédure de séparation sanguine a été précédemment mise en œuvre pour la source sanguine comprend l'attribution de l'un d'une pluralité de statuts à la source sanguine, sur la base au moins en partie de la question de savoir si la procédure de séparation sanguine a été précédemment mise en œuvre pour la source sanguine, et
chacun de la pluralité de statuts reçoit une valeur ou un poids différent lors de la détermination de la question de savoir si la source sanguine est qualifiée pour la première version ou la seconde version et/ou lors de la détermination de la question de savoir si la source sanguine n'est pas qualifiée pour la première version ou la seconde version.

5. Dispositif de séparation sanguine (10) selon l'une quelconque des revendications 2 à 4, dans lequel ledit nombre de fois que du sang a été prélevé de la source sanguine comprend le nombre de fois que du sang a été prélevé de la source sanguine dans une période de temps ou le nombre moyen de fois que du sang a été prélevé de la source sanguine dans la période de temps.

6. Dispositif de séparation sanguine (10) selon l'une quelconque des revendications 2 à 5, dans lequel l'évaluation du nombre de fois que du sang a été prélevé de la source sanguine comprend la détermination du type d'une procédure antérieure de prélèvement de sang mise en œuvre pour la source sanguine au cours de laquelle du sang a été prélevé de la source sanguine, de préférence par sélection parmi une pluralité de types de procédures de prélèvement de sang et attribution de valeurs ou de poids différents à au moins deux types différents de procédures de prélèvement de sang lors de la détermination de la question de savoir si la source sanguine est qualifiée pour la première version ou la seconde version et/ou lors de la détermination de la question de savoir si une source sanguine n'est pas qualifiée pour la première version ou la seconde version.

7. Dispositif de séparation sanguine (10) selon la revendication 6, dans lequel
un premier desdits types de procédures de prélèvement de sang est la procédure de séparation sanguine devant être mise en œuvre pour la source sanguine,
un second desdits types de procédures de prélèvement de sang n'est pas la procédure de séparation sanguine devant être mise en œuvre pour la source sanguine, et
une valeur ou un poids supérieur est attribué audit premier type de procédure de prélèvement de sang par rapport audit second type de procédure de prélèvement de sang lors de la détermination de la question de savoir si la source sanguine est qualifiée pour la première version ou la seconde version et/ou
lors de la détermination de la question de savoir si une source sanguine n'est pas qualifiée pour la première version ou la seconde version.

8. Dispositif de séparation sanguine (10) selon l'une quelconque des revendications 1 à 7, dans lequel
la procédure de séparation sanguine comprend la séparation du sang de la source sanguine en composants sanguins cellulaires et en plasma, et
une efficacité de séparation des cellules sanguines de la procédure de séparation sanguine dans la première version est différente d'une efficacité de séparation des cellules sanguines de la procédure de séparation sanguine dans la seconde version.

9. Dispositif de séparation sanguine (10) selon l'une quelconque des revendications 1 à 8, dans lequel
la procédure de séparation sanguine comprend la séparation du sang de la source sanguine en composants sanguins cellulaires et en plasma dans un circuit d'écoulement de fluide (12),
le contrôleur est en outre configuré pour déterminer ou pour recevoir un volume de globules rouges pré-procédure de la source sanguine,
un volume de globules rouges inférieur à un pourcentage sélectionné du volume de globules rouges pré-procédure est autorisé à être présent dans le circuit d'écoulement de fluide (12) à tout moment lors de la mise en œuvre de la procédure de séparation sanguine dans la première version, et
un volume de globules rouges différent dudit pourcentage sélectionné du volume de globules rouges pré-procédure est autorisé à être présent dans le circuit d'écoulement de fluide (12) à au moins un moment lors de la mise en œuvre de la procédure de séparation sanguine dans la seconde version.

10. Dispositif de séparation sanguine (10) selon l'une quelconque des revendications 1 à 8, dans lequel
la procédure de séparation sanguine comprend la séparation du sang de la source sanguine en composants sanguins cellulaires et en plasma dans un circuit d'écoulement de fluide (12),
le contrôleur est en outre configuré pour déterminer ou pour recevoir un volume sanguin total de la source sanguine,
un volume de sang inférieur à un pourcentage sélectionné du volume sanguin total est autorisé à être présent dans le circuit d'écoulement de fluide (12) à tout moment lors de la mise en œuvre de la procédure de séparation sanguine dans la première version, et
un volume de sang différent dudit pourcentage sélectionné du volume sanguin total est autorisé à être présent dans le circuit d'écoulement de fluide (12) à au moins un moment lors de la mise en œuvre de la procédure de séparation sanguine dans la seconde version.

11. Dispositif de séparation sanguine (10) selon l'une quelconque des revendications 1 à 10, dans lequel
la mise en œuvre de la procédure de séparation sanguine dans la première version comprend la séparation du sang de la source sanguine dans une première chambre de séparation sanguine (28), et
la mise en œuvre de la procédure de séparation sanguine dans la seconde version comprend la séparation du sang de la source sanguine dans une seconde chambre de séparation sanguine (28) ayant un volume différent de celui de la première chambre de séparation sanguine (28).

12. Dispositif de séparation sanguine (10) selon l'une quelconque des revendications 1 à 11, dans lequel
la mise en œuvre de la procédure de séparation sanguine dans la première version comprend l'exécution d'une première phase de prélèvement de sang ayant une durée, un débit de prélèvement et un volume de prélèvement de sang, et
la mise en œuvre de la procédure de séparation sanguine dans la seconde version comprend l'exécution d'une seconde phase de prélèvement de sang ayant une durée, un débit de prélèvement et/ou un volume de prélèvement de sang différent de ceux de ladite première phase de prélèvement de sang.

13. Dispositif de séparation sanguine (10) selon l'une quelconque des revendications 1 à 12, dans lequel
la mise en œuvre de la procédure de séparation sanguine dans la première version comprend l'exécution d'une première phase de retour de fluide ayant une durée, un débit de retour et un volume de retour de fluide, et
la mise en œuvre de la procédure de séparation sanguine dans la seconde version comprend l'exécution d'une seconde phase de retour de fluide ayant une durée, un débit de retour et/ou un volume de retour de fluide différent de ceux de ladite première phase de retour de fluide.

14. Dispositif de séparation sanguine (10) selon l'une quelconque des revendications 1 à 13, comprenant en outre un dispositif d'entrée de données (64) comprenant un bouton ou une icône (70) configuré(e) pour être manipulé(e) par un opérateur afin d'instruire le contrôleur (16) de mettre en œuvre la procédure de séparation sanguine dans la première version.

15. Dispositif de séparation sanguine (10) selon l'une quelconque des revendications 1 à 13, comprenant en outre un dispositif d'entrée de données (64) configuré pour recevoir des données saisies concernant une source sanguine, dans lequel le contrôleur (16) est configuré pour déterminer s'il convient de mettre en œuvre la procédure de séparation sanguine dans la première version par rapport à la seconde version sur la base au moins en partie desdites données saisies.
